# EUROPEAN PATENT APPLICATION

(11) **EP 4 446 737 A1**
(43) Date of publication of application: **16.10.2024**
(21) Application number: 21967055.1
(22) Date of filing: 06.12.2021
(51) Int. Cl.: G01N 33/48, G01N 33/483, G02B 21/36

(54) **CLASSIFICATION DEVICE, CLASSIFICATION METHOD, AND PROGRAM**

(71) Applicant: NEC Corporation, 108-8001 Tokyo (JP)
(72) Inventor: OMI, Yasuo, Tokyo 108-8001 (JP)
(74) Representative: Betten & Resch
(86) International application number: PCT/JP2021/044622
(87) International publication number: WO 2023/105547

(57) **Abstract**

In order to provide a technique for improving accuracy in classification of a specimen cell between benignancy and malignancy in pathological diagnosis, a classification apparatus (1) includes an acquisition section (11) for acquiring an image which includes the specimen cell as a subject; and a classification section (12) for inputting the image into a first trained model and classifying the specimen cell as a benign cell or a malignant cell based on a result of prediction, the first trained model having been trained, while using as input an image that includes a cell as a subject, so as to predict a subclass to which the cell belongs among a first benign subclass group and a first malignant subclass group.

## Description

### Technical Field

The present invention relates to a classification apparatus, a classification method, and a program for classifying a specimen cell as a benign cell or a malignant cell.

### Background Art

A technique has been disclosed in which an image of a portion to be diagnosed is captured and a feature quantity of the image is extracted.

For example, Patent Literature 1 discloses an identification apparatus which extracts a feature quantity of an image captured by imaging a human skin, and identifies whether a portion to be diagnosed in the captured image is benign or malignant with use of the feature quantity.

### Citation List

### [Patent Literature]

### [Patent Literature 1]

Japanese Patent Application Publication Tokukai No. 2021-2320

### Summary of Invention

### Technical Problem

In classification between benignancy and malignancy, for example, in a case where a false positive occurs in which classification as being malignant is made despite being benign, reinspection is needed even though reinspection is not actually unnecessary. In a case where a false negative occurs in which classification as being benign is made despite being malignant, there is also a risk of overlooking cancer. In order to reduce reinspection and overlooking, which imposes a large burden on patients, it is preferable that accuracy be high in classification between benignancy and malignancy.

An example aspect of the present invention is accomplished in view of the above problems, and an example object thereof is to provide a technique for improving accuracy in classification of a specimen cell between benignancy and malignancy in pathological diagnosis.

### Solution to Problem

A classification apparatus in accordance with an example aspect of the present invention is a classification apparatus for classifying, for pathological diagnosis, a specimen cell as a benign cell or a malignant cell, the classification apparatus including: an acquisition means for acquiring an image which includes the specimen cell as a subject; and a classification means for inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

A classification method in accordance with an example aspect of the present invention is a classification method using a classification apparatus for classifying, for pathological diagnosis, a specimen cell as a benign cell or a malignant cell, the classification method including: acquiring an image which includes the specimen cell as a subject; and inputting the image which has been acquired in the acquiring into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

A program in accordance with an example aspect of the present invention is a program for causing a computer to function as a classification apparatus for classifying, for pathological diagnosis, a specimen cell as a benign cell or a malignant cell, the program causing the computer to function as: an acquisition means for acquiring an image which includes the specimen cell as a subject; and a classification means for inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

### Advantageous Effects of Invention

According to an example aspect of the present invention, it is possible to improve accuracy in classification of a specimen cell between benignancy and malignancy in pathological diagnosis.

### Brief Description of Drawings

Fig. 1 is a block diagram illustrating a configuration of a classification apparatus in accordance with a first example embodiment of the present invention.
Fig. 2 is a flowchart illustrating a flow of a classification method in accordance with the first example embodiment of the present invention.
Fig. 3 is a block diagram illustrating a configuration of a classification apparatus in accordance with a second example embodiment of the present invention.
Fig. 4 is a table illustrating an example of classifications of a first benign subclass group and a first malignant subclass group in accordance with the second example embodiment of the present invention.
Fig. 5 is a table illustrating an example of classifications of second benign subclasses and second malignant subclasses in accordance with the second example embodiment of the present invention.
Fig. 6 is a table illustrating an example of classifications of benign cells and malignant cells in accordance with the second example embodiment of the present invention.
Fig. 7 is a flowchart illustrating a flow of a classification method in accordance with the second example embodiment of the present invention.
Fig. 8 is a block diagram illustrating an example hardware configuration of the classification apparatus in accordance with the example embodiments of the present invention.

### Example Embodiments

### [First example embodiment]

The following description will discuss a first example embodiment of the present invention in detail, with reference to the drawings. The present example embodiment is a basic form of example embodiments described later.

### (Configuration of classification apparatus 1)

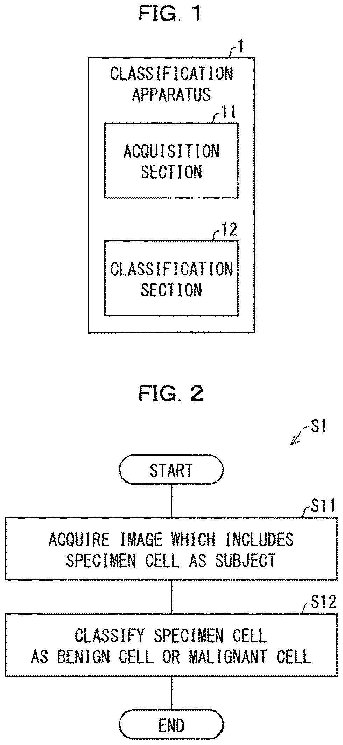

The following description will discuss a configuration of a classification apparatus 1 in accordance with the present example embodiment, with reference to Fig. 1. Fig. 1 is a block diagram illustrating a configuration of the classification apparatus 1 in accordance with the present example embodiment.

The classification apparatus 1 is an apparatus for classifying a specimen cell as a benign cell or a malignant cell (i.e., carrying out classification between benignancy and malignancy) for pathological diagnosis. For example, the classification apparatus 1 inputs an image which includes a specimen cell as a subject into a first trained model which has been trained, and classifies the specimen cell as a benign cell or a malignant cell based on a result of prediction by the first trained model.

Here, the pathological diagnosis indicates that a sample taken from a human body is observed with a microscope and diagnosis is made as to whether or not a lesion is present and as to a type of a lesion. Cytodiagnosis is a type of pathological diagnosis, and indicates carrying out classification between a benign cell and a malignant cell.

The first trained model has been trained, for example, so as to predict a subclass to which a cell included in an image as a subject belongs among a first benign subclass group in which benign cells are classified into a plurality of subclasses and a first malignant subclass group in which malignant cells are classified into a plurality of subclasses. A specific configuration of the first trained model does not limit the present example embodiment, and can be, for example, a convolution neural network (CNN), a recurrent neural network (RNN), or a combination of these networks. Alternatively, a non-neural network type model such as a random forest or a support vector machine can be used.

A subclass is obtained by classifying benign cells and malignant cells into a plurality of classifications. The first benign subclass group refers to a plurality of subclasses into which benign cells are classified, and the first malignant subclass group refers to a plurality of subclasses into which malignant cells are classified. Predicting a subclass to which a cell belongs refers to predicting to which subclass the cell belongs among the first benign subclass group and the first malignant subclass group.

That is, the first trained model is trained, upon reception of input of an image which includes a cell as a subject, to output a prediction result indicating to which subclass the cell belongs among the first benign subclass group or to which subclass the cell belongs among the first malignant subclass group.

The number of subclasses into which benign cells and malignant cells are classified is not limited. It is only necessary to employ a configuration in which benign cells and malignant cells are classified into the number of subclasses in which the first trained model can classify benign cells and malignant cells.

As illustrated in Fig. 1, the classification apparatus 1 includes an acquisition section 11 and a classification section 12. In the present example embodiment, the acquisition section 11 and the classification section 12 are components for implementing the acquisition means and the classification means, respectively.

The acquisition section 11 acquires an image which includes a specimen cell as a subject.

The classification section 12 inputs the image which has been acquired by the acquisition section 11 into a first trained model, and classifies the specimen cell as a benign cell or a malignant cell based on a result of prediction by the first trained model. For example, in a case where the result of prediction by the first trained model indicates that the specimen cell included in the image as a subject has been predicted to belong to the first benign subclass group, the classification section 12 classifies the specimen cell as a benign cell. In a case where the result of prediction by the first trained model indicates that the specimen cell included in the image as a subject has been predicted to belong to the first malignant subclass group, the classification section 12 classifies the specimen cell as a malignant cell.

As described above, the classification apparatus 1 in accordance with the present example embodiment employs the configuration of including: the acquisition section 11 for acquiring an image which includes the specimen cell as a subject; and the classification section 12 for inputting the image which has been acquired by the acquisition section 11 into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

Therefore, according to the classification apparatus 1 in accordance with the present example embodiment, the specimen cell is classified as a benign cell or a malignant cell based on a subclass to which the specimen cell included in the image as a subject belongs. In a case of a specimen cell in pathological diagnosis, signs differ depending on types of cancer, such as non-small cell carcinoma and small cell carcinoma. Therefore, the classification apparatus 1 in accordance with the present example embodiment can improve accuracy in classification of a specimen cell between benignancy and malignancy in pathological diagnosis, as compared with a case where a specimen cell included in an image as a subject is classified in a binary manner, that is, classified as a benign cell or a malignant cell.

### (Flow of classification method S1)

The following description will discuss a flow of a classification method S1 in accordance with the present example embodiment, with reference to Fig. 2. Fig. 2 is a flowchart illustrating the flow of the classification method S1 in accordance with the present example embodiment.

### (Step S11)

In step S11, the acquisition section 11 acquires an image which includes a specimen cell as a subject.

### (Step S12)

In step S12, the classification section 12 inputs the image which has been acquired in step S11 into the first trained model and classifies the specimen cell as a benign cell or a malignant cell based on a result of prediction by the first trained model, the first trained model having been trained, while using as input an image that includes a cell as a subject, so as to predict a subclass to which the cell belongs among the first benign subclass group and the first malignant subclass group.

As described above, the classification method S1 in accordance with the present example embodiment employs the configuration of including: acquiring, in step S11, an image which includes the specimen cell as a subject; and inputting, in step S12, the image which has been acquired in step S11 into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

Therefore, according to the classification method S1 in accordance with the present example embodiment, an example advantage similar to that of the classification apparatus 1 is brought about.

### [Second example embodiment]

The following description will discuss a second example embodiment of the present invention in detail, with reference to the drawings. The same reference numerals are given to constituent elements which have functions identical with those described in the first example embodiment, and descriptions as to such constituent elements are omitted as appropriate.

### (Classification apparatus 2)

A classification apparatus 2 in accordance with the present example embodiment classifies a specimen cell as a benign cell or a malignant cell for pathological diagnosis. For example, the classification apparatus 2 inputs an image (hereinafter referred to as "image PT") which includes a specimen cell as a subject into each of a plurality of trained models which have been trained, and classifies the specimen cell as a benign cell or a malignant cell based on results of prediction by the trained models. In the present example embodiment, an example will be described in which three trained models, i.e., a first trained model, a second trained model, and a third trained model are used. Note, however, that the number of trained models is not limited.

Examples of the image PT include an image which includes, as a subject, a cell of respiratory organs taken with use of an endoscope. More specifically, an image which is captured, when microscopically observing the taken cell, by a camera attached to a microscope, or an image which is sent from a microscope. The classification apparatus 2 can be used, for example, in cytodiagnosis in rapid on-site evaluation (ROSE).

As with the foregoing example embodiment, the first trained model has been trained, for example, so as to predict a subclass to which a cell included in an image PT as a subject belongs among a first benign subclass group in which benign cells are classified into a plurality of subclasses and a first malignant subclass group in which malignant cells are classified into a plurality of subclasses.

As described above, the number of subclasses into which benign cells and malignant cells are classified is not limited, and benign cells and malignant cells are classified into the number of subclasses in which the first trained model can classify benign cells and malignant cells. For example, an arbitrary subclass included in the first benign subclass group is classified so as to be different in visual sign or tissue type from the other subclasses included in the first benign subclass group. Similarly, an arbitrary subclass included in the first malignant subclass group is classified so as to be different in visual sign or tissue type from the other subclasses included in the first malignant subclass group.

The second trained model has been trained, for example, so as to predict a subclass to which a cell included in an image as a subject belongs among a second benign subclass group in which benign cells are classified into a plurality of subclasses and a second malignant subclass group in which malignant cells are classified into a plurality of subclasses.

The number of subclasses into which the second benign subclass group and the second malignant subclass group are classified is not limited, as with the first benign subclass group and the first malignant subclass group. For example, the first benign subclass group is different from the second benign subclass group, and/or the first malignant subclass group is different from the second malignant subclass group.

In the present example embodiment, a configuration will be described in which the first benign subclass group is classified into five subclasses, the second subclass group is classified into three subclasses, the first malignant subclass group is classified into four subclasses, and the second malignant subclass group is classified into two subclasses. Note, however, that these features do not limit the present example embodiment.

The third trained model has been trained, for example, so as to predict to which one of a benign cell and a malignant cell a cell included in the image PT as a subject belongs.

A specific configuration of each of the first trained model, the second trained model, and the third trained model does not limit the present example embodiment, and can be, for example, a CNN, an RNN, or a combination of these networks. Alternatively, a non-neural network type model such as a random forest or a support vector machine can be used. Training phases of the first trained model, the second trained model, and the third trained model will be described later.

### (Configuration of classification apparatus 2)

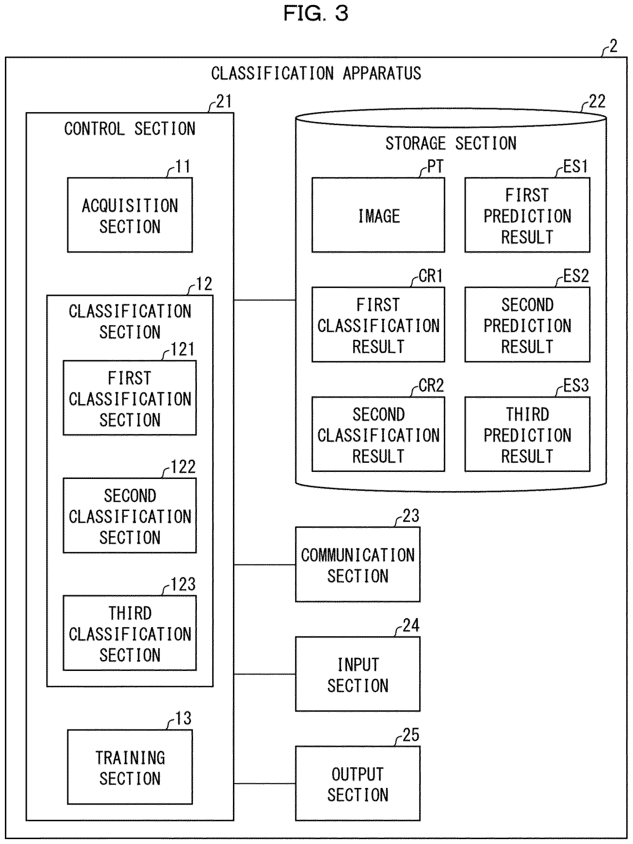

The following description will discuss a configuration of the classification apparatus 2 in accordance with the present example embodiment, with reference to Fig. 3. Fig. 3 is a block diagram illustrating a configuration of the classification apparatus 2 in accordance with the present example embodiment.

As illustrated in Fig. 3, the classification apparatus 2 includes a control section 21, a storage section 22, a communication section 23, an input section 24, and an output section 25.

In the storage section 22, data referred to by the control section 21 (described later) is stored. Examples of data stored in the storage section 22 include an image PT, a first prediction result ES1, a second prediction result ES2, a third prediction result ES3, a first classification result CR1, and a second classification result CR2. Details of these pieces of data will be described later.

The communication section 23 is a communication module that communicates with other apparatuses via a network (not illustrated). For example, the communication section 23 outputs data supplied from the control section 21 (described later) to another apparatus and acquires data output from another apparatus via a network and supplies the data to the control section 21.

The input section 24 is an interface via which data is acquired from another apparatus which is connected to the classification apparatus 2. The input section 24 supplies data acquired from another apparatus to the control section 21 (described later).

The output section 25 is an interface via which data is outputted to another apparatus which is connected to the classification apparatus 2. The output section 25 outputs data supplied from the control section 21 (described later) to another apparatus.

### (Control section 21)

The control section 21 controls components included in the classification apparatus 2. For example, the control section 21 causes the storage section 22 to store data acquired from the communication section 23 or the input section 24 and supplies data stored in the storage section 22 to the communication section 23 or the output section 25.

As illustrated in Fig. 3, the control section 21 functions also as an acquisition section 11, a classification section 12, and a training section 13. In the present example embodiment, the acquisition section 11, the classification section 12, and the training section 13 are components for implementing the acquisition means, the classification means, and the training means, respectively.

The acquisition section 11 acquires data supplied from the communication section 23 or the input section 24 and causes the storage section 22 to store the acquired data. Examples of data acquired by the acquisition section 11 include an image PT and a second classification result CR2 associated with the image PT. The acquisition section 11 causes the storage section 22 to store the acquired data. The image PT is as described above. The second classification result CR2 will be described later.

The classification section 12 acquires the image PT which is stored in the storage section 22, and classifies, as a benign cell or a malignant cell, a cell included in the acquired image PT as a subject. The classification section 12 causes the storage section 22 to store a first classification result CR1 indicating the result of classification.

The classification section 12 carries out classification based on, for example, the following results of prediction.
- A first prediction result ES1 that has been obtained by inputting an image PT into the first trained model and that indicates to which one of the first benign subclass group and the first malignant subclass group a cell included in the image PT as a subject belongs.
- A second prediction result ES2 that has been obtained by inputting an image PT into the second trained model and that indicates to which one of the second benign subclass group and the second malignant subclass group a cell included in the image PT as a subject belongs.
- A third prediction result ES3 that has been obtained by inputting an image PT into the third trained model and that indicates whether a cell included in the image PT as a subject has been predicted to be a benign cell or a malignant cell.

For example, in a case where at least one of the prediction results output respectively from the first trained model, the second trained model, and the third trained model (plurality of trained models) indicates that the specimen cell has been classified into the benign subclass group or as a benign cell, the classification section 12 classifies the specimen cell as a benign cell. In other words, in a case where prediction results indicating that the specimen cell has been classified into the malignant subclass group or as a malignant cell have been obtained from all the plurality of trained models, the classification section 12 classifies the specimen cell as a malignant cell.

With this configuration, the classification apparatus 2 can reduce a possibility that a false positive occurs in which classification as being a malignant cell is made despite being a benign cell. The classification apparatus 2 can reduce the burden on a patient due to reinspection by reducing the possibility that false positives will occur.

For another example, the classification section 12 can be configured to employ a prediction result of the larger one of the number of prediction results indicating that the specimen cell has been classified into the benign subclass group or as a benign cell and the number of prediction results indicating that the specimen cell has been classified into the malignant subclass group or as a malignant cell.

For example, prediction results are as follows:
- the first prediction result ES1 indicates that the specimen cell has been predicted to belong to the first benign subclass group;
- the second prediction result ES2 indicates that the specimen cell has been predicted to belong to the second malignant subclass group; and
- the third prediction result ES3 indicates that the specimen cell has been predicted to be a malignant cell.
In such a case, the number of prediction results indicating that the specimen cell has been classified into the benign subclass group or as a benign cell is 1, and the number of prediction results indicating that the specimen cell has been classified into the malignant subclass group or as a malignant cell is 2. Therefore, the classification section 12 classifies the specimen cell as a malignant cell.

As illustrated in Fig. 3, the classification section 12 functions also as a first classification section 121, a second classification section 122, and a third classification section 123.

The first classification section 121 inputs an image PT into the first trained model and acquires a first prediction result ES 1.

The second classification section 122 inputs the image PT into the second trained model and acquires a second prediction result ES2.

The third classification section 123 inputs the image PT into the third trained model and acquires a third prediction result ES3.

The training section 13 acquires training data that is stored in the storage section 22 and that includes a set of an image PT and a second classification result CR2 associated with the image PT, and trains at least one of the first trained model, the second trained model, and the third trained model with use of the training data. Here, the second classification result CR2 is a correct answer label associated with the image PT. In other words, the second classification result CR2 is information indicating a subclass to which a cell included in the image PT belongs. The following description will discuss an example of a training phase.

### (Example 1 of training phase)

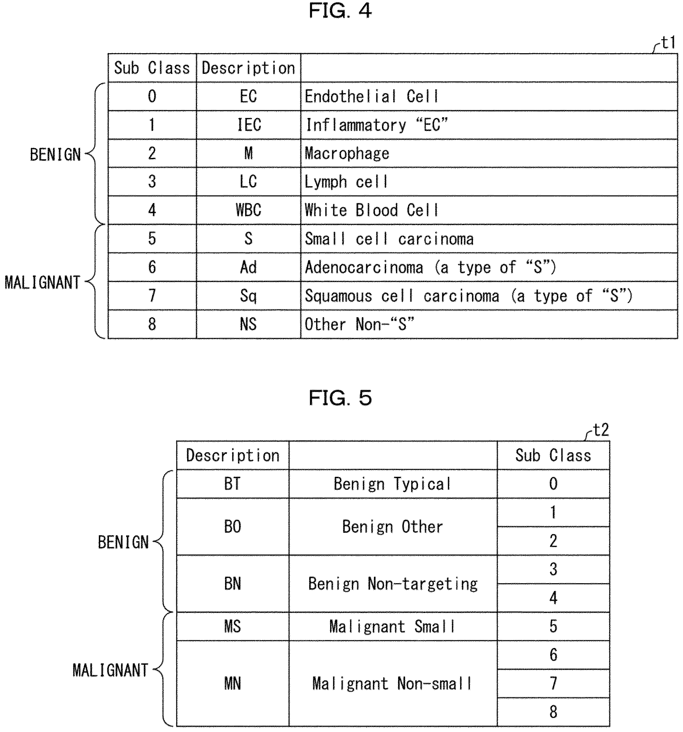

The following description will discuss a training phase of the first trained model with reference to Fig. 4. Fig. 4 is a table t1 illustrating an example of classifications of a first benign subclass group and a first malignant subclass group in accordance with the present example embodiment.

The table t1 illustrated in Fig. 4 indicates a first benign subclass group which is classified into five subclasses and a first malignant subclass group which is classified into four subclasses. As illustrated in Fig. 4, in the table t1, subclass numbers "0" through "4" are associated with the five subclasses, respectively, included in the first benign subclass group. For example, the subclass number "0" is associated with a subclass "EC" among the first benign subclass group.

Similarly, in the table t1, subclass numbers "5" through "8" are associated with the four subclasses, respectively, included in the first malignant subclass group. For example, the subclass number "5" is associated with a subclass "S" among the first malignant subclass group.

The training section 13 trains the first trained model with use of the table t1 and training data that includes a set of an image PT and a second classification result CR2 indicating a subclass to which a cell included in the image PT as a subject belongs.

For example, in a case where the acquisition section 11 has acquired training data of a set of an image PT and a second classification result CR2 indicating that a cell included in the image PT as a subject belongs to the subclass number "0", the training section 13 first inputs the image PT into the first trained model. Then, in a case where a first prediction result ES1 output from the first trained model is not "EC" associated with the subclass number "0" in the table t1, the training section 13 updates parameters of the first trained model so that a first prediction result ES1 indicating "EC" is output in a case where the image PT is input.

### (Example 2 of training phase)

The following description will discuss a training phase of the second trained model with reference to Fig. 5. Fig. 5 is a table t2 illustrating an example of classifications of second benign subclasses and second malignant subclasses in accordance with the present example embodiment.

The table t2 illustrated in Fig. 5 indicates a second benign subclass group which is classified into three subclasses and a second malignant subclass group which is classified into two subclasses. The subclass numbers indicated in the table t2 are identical with those in the table t1.

As illustrated in Fig. 5, in the table t2, one or more subclass numbers are associated with each of the three subclasses included in the first benign subclass group. For example, the subclass number "0" is associated with a subclass "BT" among the second benign subclass group. For another example, the subclass numbers "1" and "2" are associated with a subclass "BO" among the second benign subclass group.

Similarly, in the table t2, one or more subclass numbers are associated with each of the two subclasses included in the second malignant subclass group. For example, the subclass number "5" is associated with a subclass "MS" among the second malignant subclass group. For another example, the subclass numbers "6", "7", and "8" are associated with a subclass "MN" among the second malignant subclass group.

The training section 13 trains the second trained model with use of the table t2 and training data that includes a set of an image PT and a second classification result CR2 indicating a subclass to which a cell included in the image PT as a subject belongs.

For example, in a case where the acquisition section 11 has acquired training data of a set of an image PT and a second classification result CR2 indicating that a cell included in the image PT as a subject belongs to the subclass number "3", the training section 13 first inputs the image PT into the second trained model. Then, in a case where a second prediction result ES2 output from the second trained model is not "BN" associated with the subclass number "3" in the table t2, the training section 13 updates parameters of the second trained model so that a second prediction result ES2 indicating "BN" is output in a case where the image PT is input.

### (Example 3 of training phase)

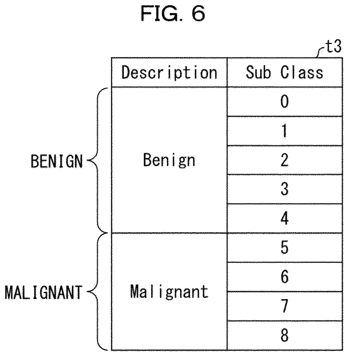

The following description will discuss a training phase of the third trained model with reference to Fig. 6. Fig. 6 is a table t3 illustrating an example of classifications of benign cells and malignant cells in accordance with the present example embodiment.

The table t3 illustrated in Fig. 6 indicates whether each subclass belongs to a benign cell or a malignant cell. As illustrated in Fig. 6, subclass numbers "0" through "4" are associated with "Benign" indicating that the subclass is a benign cell, and subclass numbers "5" through "8" are associated with "Malignant" indicating that the subclass is a malignant cell. As with the table t2, the subclass numbers indicated in the table t3 are also identical with those in the table t1.

The training section 13 trains the third trained model with use of the table t3 and training data that includes a set of an image PT and a second classification result CR2 indicating a subclass to which a cell included in the image PT as a subject belongs.

For example, in a case where the acquisition section 11 has acquired training data of a set of an image PT and a second classification result CR2 indicating that a cell included in the image PT as a subject belongs to the subclass number "5", the training section 13 first inputs the image PT into the third trained model. Then, in a case where a third prediction result ES3 output from the third trained model is not "Malignant" associated with the subclass number "5" in the table t3, the training section 13 updates parameters of the third trained model so that a third prediction result ES3 indicating "Malignant" is output in a case where the image PT is input.

### (Flow of classification method S2)

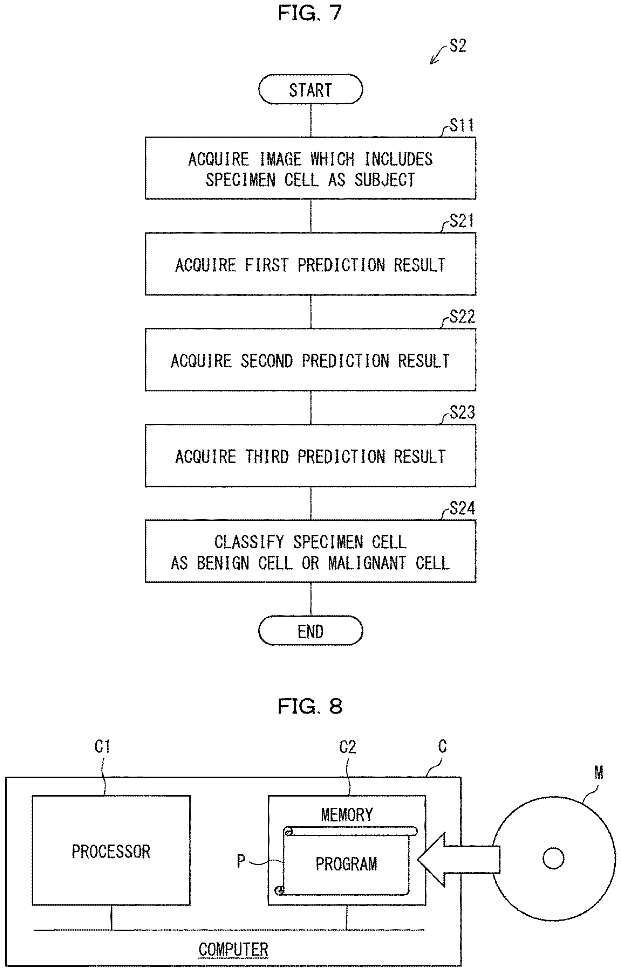

The following description will discuss a flow of a classification method S2 in accordance with the present example embodiment, with reference to Fig. 7. Fig. 7 is a flowchart illustrating the flow of the classification method S2 in accordance with the present example embodiment.

### (Step S11)

In step S11, the acquisition section 11 acquires an image PT which includes a specimen cell as a subject. The acquisition section 11 causes the storage section 22 to store the acquired image PT.

### (Step S21)

In step S21, the first classification section 121 of the classification section 12 acquires the image PT from the storage section 22. The first classification section 121 inputs the acquired image PT into the first trained model and acquires a first prediction result ES1. The first classification section 121 causes the storage section 22 to store the acquired first prediction result ES1.

### (Step S22)

In step S22, the second classification section 122 of the classification section 12 acquires the image PT from the storage section 22. The second classification section 122 inputs the acquired image PT into the second trained model and acquires a second prediction result ES2. The second classification section 122 causes the storage section 22 to store the acquired second prediction result ES2.

### (Step S23)

In step S23, the third classification section 123 of the classification section 12 acquires the image PT from the storage section 22. The third classification section 123 inputs the acquired image PT into the third trained model and acquires a third prediction result ES3. The third classification section 123 causes the storage section 22 to store the acquired third prediction result ES3.

### (Step S24)

In step S24, the classification section 12 acquires, from the storage section 22, the first prediction result ES1, the second prediction result ES2, and the third prediction result ES3. The classification section 12 classifies, as a benign cell or a malignant cell, a specimen cell included in the image PT as a subject with reference to the first prediction result ES1, the second prediction result ES2, and the third prediction result ES3. An example in which the classification section 12 classifies the specimen cell as a benign cell or a malignant cell is as described above.

As described above, the classification apparatus 2 in accordance with the present example embodiment classifies a specimen cell, which is included in the image PT as a subject, as a benign cell or a malignant cell based on the first prediction result ES1, the second prediction result ES2, and the third prediction result ES3 output from the first trained model, the second trained model, and the third trained model, respectively.

Therefore, according to the classification apparatus 2 in accordance with the present example embodiment, the specimen cell is classified as a benign cell or a malignant cell based on a plurality of prediction results. Therefore, it is possible to improve accuracy in classification of a specimen cell between benignancy and malignancy in pathological diagnosis. Further, according to the classification apparatus 2 in accordance with the present example embodiment, the specimen cell is classified as a benign cell or a malignant cell based on a plurality of prediction results. Therefore, it is possible to reduce a possibility that false positives occur.

As described above, in the classification apparatus 2 in accordance with the present example embodiment, the number of trained models is not limited. For example, the classification apparatus 2 can be configured to use two trained models among the first trained model, the second trained model, and the third trained model. For another example, the classification apparatus 2 may be configured to use a fourth trained model in addition to the first trained model, the second trained model, and the third trained model. The fourth trained model classifies the specimen cell into a fourth benign subclass group which is different from the first benign subclass group and the second benign subclass group and a fourth malignant subclass group which is different from the first malignant subclass group and the second malignant subclass group.

### [Third example embodiment]

The following description will discuss a third example embodiment of the present invention. The same reference numerals are given to constituent elements which have functions identical with those described in the foregoing example embodiments, and descriptions as to such constituent elements are omitted as appropriate.

### (Classification apparatus 3)

The classification apparatus 3 in accordance with the present example embodiment may be configured such that the first trained model, the second trained model, and the third trained model in the classification apparatus 2 in accordance with the second example embodiment described above are implemented by a single trained model. The classification apparatus 3 in accordance with the present example embodiment may be configured to use any of the first trained model, the second trained model, and the third trained model in the classification apparatus 2.

The classification apparatus 3 inputs an image PT stored in the storage section 22 into the trained model, and acquires a prediction result ES from the trained model. Then, the classification section 12 classifies, with reference to the prediction result ES, a specimen cell, which is included in the image PT as a subject, as a benign cell or a malignant cell. An example in which the classification section 12 classifies the specimen cell as a benign cell or a malignant cell is as described above.

As described above, the classification apparatus 3 in accordance with the present example embodiment inputs the image PT into the trained model, and then, with reference to the prediction result ES acquired from the trained model, classifies the specimen cell, which is included in the image PT as a subject, as a benign cell or a malignant cell. As described above, the classification apparatus 3 in accordance with the present example embodiment can improve accuracy in classification of a specimen cell between benignancy and malignancy in pathological diagnosis.

### [Software implementation example]

Some or all of the functions of each of the classification apparatuses 1, 2, and 3 may be implemented by hardware such as an integrated circuit (IC chip), or may be implemented by software.

In the latter case, the classification apparatuses 1, 2, and 3 are implemented by, for example, a computer that executes instructions of a program that is software implementing the foregoing functions. Fig. 8 illustrates an example of such a computer (hereinafter, referred to as "computer C"). The computer C includes at least one processor C 1 and at least one memory C2. The memory C2 stores a program P for causing the computer C to operate as the classification apparatuses 1, 2, and 3. The processor C1 of the computer C retrieves the program P from the memory C2 and executes the program P, so that the functions of the classification apparatuses 1, 2, and 3 are implemented.

As the processor C1, for example, it is possible to use a central processing unit (CPU), a graphic processing unit (CPU), a digital signal processor (DSP), a micro processing unit (MPU), a floating point number processing unit (FPU), a physics processing unit (PPU), a microcontroller, or a combination of these. Examples of the memory C2 include a flash memory, a hard disk drive (HDD), a solid state drive (SSD), and a combination thereof.

Note that the computer C can further include a random access memory (RAM) in which the program P is loaded when the program P is executed and in which various kinds of data are temporarily stored. The computer C can further include a communication interface for carrying out transmission and reception of data with other apparatuses. The computer C can further include an input-output interface for connecting input-output apparatuses such as a keyboard, a mouse, a display and a printer.

The program P can be stored in a computer C-readable, non-transitory, and tangible storage medium M. The storage medium M can be, for example, a tape, a disk, a card, a semiconductor memory, a programmable logic circuit, or the like. The computer C can obtain the program P via the storage medium M. The program P can be transmitted via a transmission medium. The transmission medium can be, for example, a communications network, a broadcast wave, or the like. The computer C can obtain the program P also via such a transmission medium.

### [Additional remark 1]

The present invention is not limited to the foregoing example embodiments, but may be altered in various ways by a skilled person within the scope of the claims. For example, the present invention also encompasses, in its technical scope, any example embodiment derived by appropriately combining technical means disclosed in the foregoing example embodiments.

### [Additional remark 2]

Some or all of the foregoing example embodiments can also be described as below. Note, however, that the present invention is not limited to the following supplementary notes.

### (Supplementary note 1)

A classification apparatus for classifying, for pathological diagnosis, a specimen cell as a benign cell or a malignant cell, the classification apparatus including: an acquisition means for acquiring an image which includes the specimen cell as a subject; and a classification means for inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

According to the above configuration, it is possible to improve accuracy in classification of a specimen cell between benignancy and malignancy in pathological diagnosis.

### (Supplementary note 2)

The classification apparatus according to supplementary note 1, in which: an arbitrary subclass included in the first benign subclass group differs in visual sign or tissue type from the other subclasses included in the first benign subclass group; and an arbitrary subclass included in the first malignant subclass group differs in visual sign or tissue type from the other subclasses included in the first malignant subclass group.

According to the above configuration, it is possible to carry out classification into possible subclasses.

### (Supplementary note 3)

The classification apparatus according to supplementary note 1 or 2, in which: the classification means further inputs the image which has been acquired by the acquisition means into a second trained model which has been trained, while using as input an image that includes a cell as a subject, so as to predict a subclass to which the cell belongs among a second benign subclass group in which benign cells are classified into a plurality of subclasses and a second malignant subclass group in which malignant cells are classified into a plurality of subclasses; the classification means classifies the specimen cell as a benign cell or a malignant cell based further on a result of prediction by the second trained model; and the first benign subclass group differs from the second benign subclass group and/or the first malignant subclass group differs from the second malignant subclass group.

According to the above configuration, it is possible to improve accuracy in classification of a specimen cell between benignancy and malignancy in pathological diagnosis.

### (Supplementary note 4)

The classification apparatus according to any one of supplementary notes 1 through 3, in which: the classification means further inputs the image which has been acquired by the acquisition means into a third trained model which has been trained, while using as input an image that includes a cell as a subject, so as to predict to which one of a benign cell and a malignant cell the cell belongs; and the classification means classifies the specimen cell as a benign cell or a malignant cell based further on a result of prediction by the third trained model.

According to the above configuration, it is possible to improve accuracy in classification of a specimen cell between benignancy and malignancy in pathological diagnosis.

### (Supplementary note 5)

The classification apparatus according to supplementary note 4, in which: in a case where at least one of the results output from the respective plurality of trained models indicates that the specimen cell has been classified into the benign subclass group or as a benign cell, the classification means classifies the specimen cell as a benign cell.

According to the above configuration, it is possible to reduce a possibility that false positives occur.

### (Supplementary note 6)

The classification apparatus according to any one of supplementary notes 1 through 5, in which: the acquisition means further acquires training data including a set of an image which includes a cell as a subject and a subclass to which the cell belongs; and the classification apparatus further includes a training means for training the trained model with use of the training data which has been acquired by the acquisition means.

According to the above configuration, it is possible to train the trained model.

### (Supplementary note 7)

The classification apparatus according to any one of supplementary notes 1 through 6, in which: the acquisition means acquires, when microscopically observing a cell of respiratory organs taken with used of an endoscope, an image captured by a camera attached to a microscope.

According to the above configuration, it is possible to use the classification apparatus for cytodiagnosis in ROSE.

### (Supplementary note 8)

A classification method using a classification apparatus for classifying, for pathological diagnosis, a specimen cell as a benign cell or a malignant cell, the classification method including: acquiring an image which includes the specimen cell as a subject; and inputting the image which has been acquired in the acquiring into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

According to the above configuration, it is possible to improve accuracy in classification of a specimen cell between benignancy and malignancy in pathological diagnosis.

### (Supplementary note 9)

A program for causing a computer to function as a classification apparatus for classifying, for pathological diagnosis, a specimen cell as a benign cell or a malignant cell, the program causing the computer to function as: an acquisition means for acquiring an image which includes the specimen cell as a subject; and a classification means for inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

According to the above configuration, it is possible to improve accuracy in classification of a specimen cell between benignancy and malignancy in pathological diagnosis.

### [Additional remark 3]

Some or all of the foregoing example embodiments can also be described as below.

A classification apparatus for classifying, for pathological diagnosis, a specimen cell as a benign cell or a malignant cell, the classification apparatus including at least one processor, the at least one processor carrying out: an acquisition process of acquiring an image which includes the specimen cell as a subject; and a classification process of inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

Note that the classification apparatus can further include a memory. The memory can store a program for causing the at least one processor to execute the acquisition process and the classification process. The program can be stored in a computer-readable non-transitory tangible storage medium.

### Reference Signs List

1, 2: Classification apparatus
11: Acquisition section
12: Classification section
13: Training section
21: Control section
22: Storage section
23: Communication section
24: Input section
25: Output section
121: First classification section
122: Second classification section
123: Third classification section

## Claims

1. A classification apparatus for classifying, for pathological diagnosis, a specimen cell as a benign cell or a malignant cell, said classification apparatus comprising:
an acquisition means for acquiring an image which includes the specimen cell as a subject; and
a classification means for inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

2. The classification apparatus according to claim 1, wherein:
an arbitrary subclass included in the first benign subclass group differs in visual sign or tissue type from the other subclasses included in the first benign subclass group; and
an arbitrary subclass included in the first malignant subclass group differs in visual sign or tissue type from the other subclasses included in the first malignant subclass group.

3. The classification apparatus according to claim 1 or 2, wherein:
the classification means further inputs the image which has been acquired by the acquisition means into a second trained model which has been trained, while using as input an image that includes a cell as a subject, so as to predict a subclass to which the cell belongs among a second benign subclass group in which benign cells are classified into a plurality of subclasses and a second malignant subclass group in which malignant cells are classified into a plurality of subclasses;
the classification means classifies the specimen cell as a benign cell or a malignant cell based further on a result of prediction by the second trained model; and
the first benign subclass group differs from the second benign subclass group and/or the first malignant subclass group differs from the second malignant subclass group.

4. The classification apparatus according to any one of claims 1 through 3, wherein:
the classification means further inputs the image which has been acquired by the acquisition means into a third trained model which has been trained, while using as input an image that includes a cell as a subject, so as to predict to which one of a benign cell and a malignant cell the cell belongs; and
the classification means classifies the specimen cell as a benign cell or a malignant cell based further on a result of prediction by the third trained model.

5. The classification apparatus according to claim 4, wherein:
in a case where at least one of the results output from the respective plurality of trained models indicates that the specimen cell has been classified into the benign subclass group or as a benign cell, the classification means classifies the specimen cell as a benign cell.

6. The classification apparatus according to any one of claims 1 through 5, wherein:
the acquisition means further acquires training data including a set of an image which includes a cell as a subject and a subclass to which the cell belongs; and
said classification apparatus further comprises a training means for training the trained model with use of the training data which has been acquired by the acquisition means.

7. The classification apparatus according to any one of claims 1 through 6, wherein:
the acquisition means acquires, when microscopically observing a cell of respiratory organs taken with used of an endoscope, an image captured by a camera attached to a microscope.

8. A classification method using a classification apparatus for classifying, for pathological diagnosis, a specimen cell as a benign cell or a malignant cell, said classification method comprising:
acquiring an image which includes the specimen cell as a subject; and
inputting the image which has been acquired in the acquiring into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.

9. A program for causing a computer to function as a classification apparatus for classifying, for pathological diagnosis, a specimen cell as a benign cell or a malignant cell, said program causing the computer to function as:
an acquisition means for acquiring an image which includes the specimen cell as a subject; and
a classification means for inputting the image which has been acquired by the acquisition means into a first trained model and classifying the specimen cell as a benign cell or a malignant cell based on a result of prediction by the first trained model, the first trained model having been trained, while using as input an image that includes a cell as a subject, so as to predict a subclass to which the cell belongs among a first benign subclass group in which benign cells are classified into a plurality of subclasses and a first malignant subclass group in which malignant cells are classified into a plurality of subclasses.
